# EUROPÄISCHE PATENTANMELDUNG

(11) **EP 1 279 669 A2**
(43) Veröffentlichungstag der Anmeldung: **29.01.2003**
(21) Anmeldenummer: 02090243.3
(22) Anmeldetag: 11.07.2002
(51) Int. Cl.: C07D 251/00, C08G 12/00

(54) **Polyalkylenoxid-freie Gemische aus Triazinderivaten**

(30) Priorität: 26.07.2001 DE 10136322
(71) Anmelder: Agrolinz Melamin GmbH, A-4021 Linz (AT)
(72) Erfinder: Rätzsch, Manfred, Prof. Dr., 4062 Wilhering (AT); Burger, Martin, Dr., 4020 Linz (AT); Arnold, Manfred Prof. Dr., 06667 Leissling (DE); Frank, Willy, Dr., 06246 Bad Lauchstädt (DE)
(74) Vertreter: Wablat, Wolfgang, Dr.Dr.

(57) **Zusammenfassung**

Polyalkylenoxid-freie Gemische aus 20 bis 70 Masse% Triazinderivaten mit Hydroxyalkylaminogruppen und 80 bis 30 Masse% Triazinderivaten mit Bis(hydroxyalkyl)iminogruppen werden durch katalytische Umsetzung von Alkylenoxiden mit Triazinderivaten bei einem Molverhältnis Melaminderivat/Alkylenoxid 1 : 1,5 bis 1 : 10 in Gegenwart von Metallalkylen, Metallhydriden, Metallalkoholaten und/oder Metallalkylamiden als Katalysatoren hergestellt.

Die Polyalkylenoxid-freien Gemische aus Triazinderivaten mit Hydroxygruppen sind zur Herstellung von Kunststoffen, Flammschutzmitteln, Additiven, Pharmazeutika, Textilhilfsmitteln und Farbstoffen geeignet.

## Beschreibung

Die Erfindung betrifft Polyalkylenoxid-freie Gemische aus Triazinderivaten mit Hydroxygruppen sowie ein Verfahren zu deren Herstellung.

Triazinderivate mit Hydroxygruppen von Typ Methylolmelamine oder Methylolguanamine, die durch Umsetzung von Melamin bzw. Guanamin mit Formaldehyd hergestellt werden können, sind bekannt [Ullmanns Encyclopedia of Industrial Chemistry (1987), Vol. A2, 130-131; Duroplaste, Kunststoff-Handbuch Bd. 10, 994-997, Carl Hanser-Verlag München 1988]. Von Nachteil bei diesen Methylolmelaminen oder Methylolguanaminen ist deren begrenzte Stabilität, die zu einer partiellen Rückspaltung in Melamin bzw. Guanamin und Formaldehyd führen kann.

Bekannt sind weiterhin Hydroxyoxaalkylmelamine, bei denen die Aminogruppe(n) des Melamins durch Gruppen der Formel H-(-O-CHR'-CHR'-)ₙ substituiert sind, wobei R' = C₁-C₄-Alkylgruppen sind und n=2 oder 3 bedeuten und die Herstellung der Hydroxyoxaalkylmelamine durch Umsetzung von Aminoethoxyethanol mit Cyanursäurechlorid, Dichloraminotriazin oder Diaminochlortriazin erfolgt (EP 0 225 433). Neben dem aufwendigen Herstellungsverfahren für Hydroxyoxaalkylmelamine besteht der Nachteil darin, dass sich nach diesem Herstellungsverfahren Hydroxyalkylmelamine mit n=1 nicht herstellen lassen.

Ebenfalls bekannt ist die Umsetzung von Melamin mit Ethylenoxid in Gegenwart von Alkalimetallhydroxiden (DE 21 18 868) oder von N.N'-Hydroxyethylmelaminen oder N.N'-Hydroxyarylmelaminen mit Ethylenoxid oder Propylenoxid in Gegenwart von Schwefelsäure (US 4 356 304). Der Nachteil dieser Alkylenoxid-modifizierten Melamine als Melamin-Alkylenoxid-Blockcopolymere besteht in den langen Polyalkylenoxidsequenzen und in den störenden Anteilen an nichtgebundenem Polyalkylenoxid.

Ziel der Erfindung sind Triazinderivate mit Hydroxygruppen, die einen hohen Anteil an Hydroxyalkylgruppen und keine Anteile an Polyalkylenoxiden besitzen.

Die Aufgabe der Erfindung wurde durch Polyalkylenoxid-freie Gemische aus Triazinderivaten mit Hydroxygruppen gelöst, wobei die Gemische aus
a) 20 bis 70 Masse% Triazinderivaten mit Hydroxyalkylaminogruppen der Formel 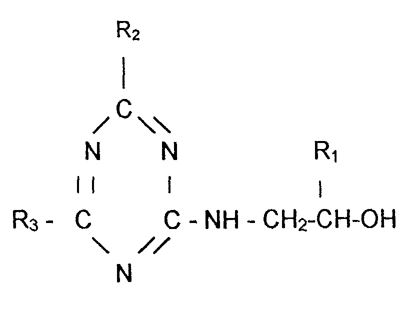
   R₁ = H oder C₁-C₄ - Alkyl
   R₂ = -H; -OH; C₁-C₄ - Alkyl, -C₆H₅; -NH₂; -NH-CH₂-CHR₁-OH; -NH-CH₂-OR₁ oder -N(-CH₂-OR₁)₂,
   R₃ = -H; -OH; C₁-C₄ - Alkyl, -C₆H₅; -NH₂; -NH-CH₂-CHR₁-OH; -NH-CH₂-OR₁ oder -N(-CH₂-OR₁)₂,
   und
b) 80 bis 30 Masse% Triazinderivaten mit Bis(hydroxyalkyl)iminogruppen der Formel 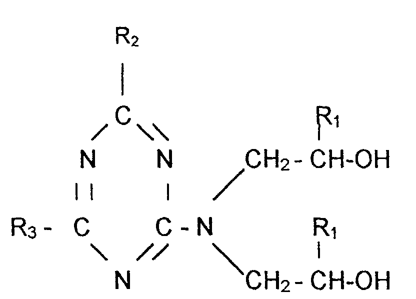
   R₁ = H oder C₁-C₄ - Alkyl
   R₂ = -H; -OH; C₁-C₄ - Alkyl, -C₆H₅; -NH₂; -NH-CH₂-CHR₁-OH; -N[CH₂-CHR₁-OH]₂, -NH-CH₂-OR₁ oder -N(-CH₂-OR₁)₂,
   R₃ = -H; -OH; C₁-C₄ - Alkyl, -C₆H₅; -NN₂; -NH-CH₂-CHR₁-OH; -N[CH₂-CHR₁-OH]₂, -NH-CH₂-OR₁ oder -N(-CH₂-OR₁)₂,
   bestehen.

Beispiele für Triazinderivate, die erfindungsgemäss sowohl durch Hydroxyalkylaminogruppen als auch durch Bis(hydroxyalkyl)iminogruppen substituiert sind, sind Melamine, Amino-C₁-C₁₂-alkylgruppen substituierte Melamine, Diaminomethyltriazine wie 2,4-Di(6-aminohexylamino)-1,3,5-triazin oder Diaminophenyltriazine, veretherte Methylolmelamine, Ammeline, Ammelide, Benzoguanamine, Acetoguanamine, Methoxymethylbenzoguanamine, Caprinoguanamine und Butyroguanamine, deren Aminogruppe(n) sowohl durch Hydroxyalkylaminogruppen als auch durch Bis(hydroxyalkyl)iminogruppen substituiert sind,

Bevorzugt werden als Polyalkylenoxid-freie Gemische aus Triazinderivaten mit Hydroxygruppen, bei denen die Gemische aus
a) Hydroxyalkylaminogruppen substituiertem Triazin mit einem Molverhältnis Triazin/ Hydroxyalkylaminogruppen 1 : 1 bis 1 : 2,5, und
b) Bis(hydroxyalkyl)iminogruppen und Hydroxyalkylaminogruppen substituiertem Triazin mit einem Molverhältnis Triazin/Hydroxyalkylgruppen 1 : 2,5 bis 1 : 5,5
bestehen.

Beispiele für Hydroxypropylaminogruppen- bzw. Bis(hydroxyalkyl)iminogruppen substituierte Melamine sind Melaminderivate der Struktur:

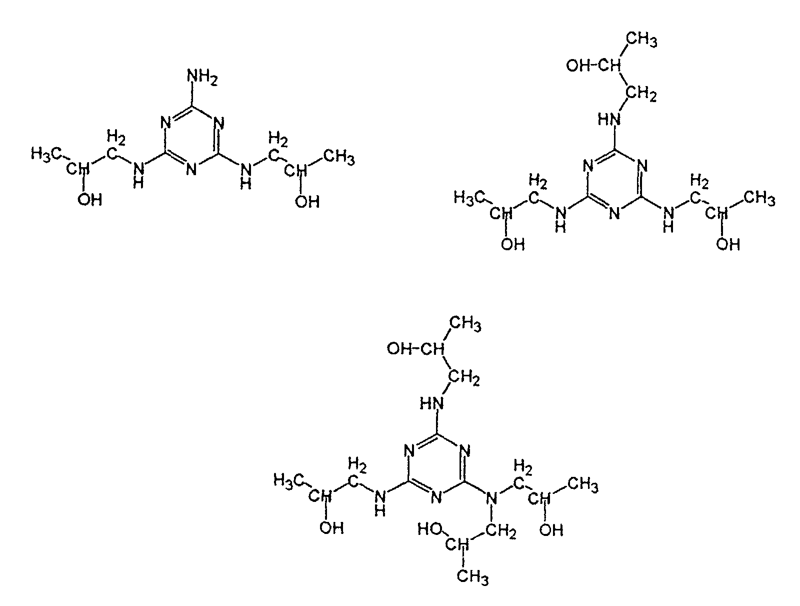

Die Polyalkylenoxid-freien Gemische aus Triazinderivaten mit Hydroxygruppen werden erfindungsgemäss nach einem Verfahren hergestellt, wobei Gemische, die aus
a) 20 bis 70 Masse% Triazinderivaten mit Hydroxyalkylaminogruppen der Formel

a) 20 bis 70 Masse% Triazinderivaten mit Hydroxyalkylaminogruppen der Formel 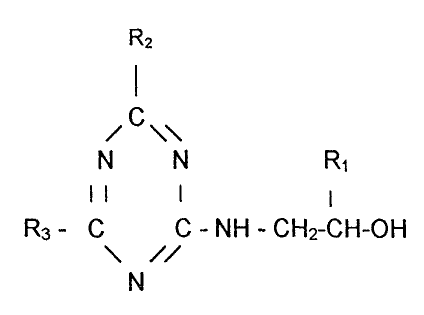
   R₁ = H oder C₁-C₄ - Alkyl
   R₂ = -H; -OH; C₁-C₄ - Alkyl, -C₆H₅; -NH₂; -NH-CH₂-CHR₁-OH; -NH-CH₂-OR₁ oder -N(-CH₂-OR₁)₂,
   R₃ = -H; -OH; C₁-C₄ - Alkyl, -C₆H₅; -NH₂; -NH-CH₂-CHR₁-OH; -NH-CH₂-OR₁ oder -N(-CH₂-OR₁)₂,
   und
b) 80 bis 30 Masse% Triazinderivaten mit Bis(hydroxyalkyl)iminogruppen der Formel 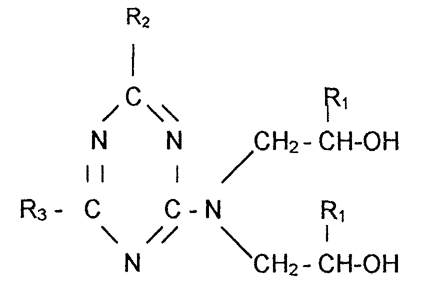
   R₁ = H oder C₁-C₄ - Alkyl
   R₂ = -H; -OH; C₁-C₄ - Alkyl, -C₆H₅; -NH₂; -NH-CH₂-CHR₁-OH; -N[CH₂-CHR₁-OH]₂, -NH-CH₂-OR₁ oder -N(-CH₂-OR₁)₂,
   R₃ = -H; -OH; C₁-C₄ - Alkyl, -C₆H₅; -NH₂; -NH-CH₂-CHR₁-OH; -N[CH₂-CHR₁-OH]₂, -NH-CH₂-OR₁ oder -N(-CH₂-OR₁)₂,
   bestehen, durch katalytische Umsetzung von Alkylenoxiden der Formel 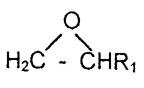 , wobei R₁ = H oder C₁-C₄ - Alkyl bedeuten,
   mit Triazinderivaten der Formel 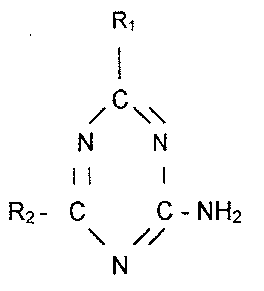
   R₁ = -H; -OH; C₁-C₄ - Alkyl, -C₆H₅; -NH₂; -NH-CH₂-OR₁ oder -N(-CH₂-OR₁)₂,
   R₂ = -H; -OH; C₁-C₄ - Alkyl, -C₆H₅; -NH₂; -NH-CH₂-OR₁ oder -N(-CH₂-OR₁)₂,
bei einem Molverhältnis Triazinderivat/Alkylenoxid 1 : 1,5 bis 1 : 10 in Gegenwart von 0,001 bis 1 Masse%, bezogen auf die Summe von Triazinderivat und Alkylenoxid, Metallalkylen, Metallhydriden, Metallalkoholaten und/oder Metallalkylamiden als Katalysatoren hergestellt werden, wobei die Umsetzung in polaren wasserfreien Lösungsmitteln vom Typ Dimethylsulfoxid, Tetramethylensulfon, N-Methylpyrrolidinon, Dimethylformamid und/oder Dimethylacetamid bei Reaktionstemperaturen von 35 bis 150°C, Drücken von 1 bis 25 bar und Reaktionszeiten von 1 bis 30 Std. durchgeführt wird.

Geeignete Reaktoren für die Umsetzung der Triazinderivate mit Alkylenoxiden sind Rührreaktoren mit Inertgasbeaufschlagung, absteigendem Kühler und Bodenablassventil.

Nach der Umsetzung ist es von Vorteil, die Aufarbeitung des Reaktionsgemisches durch Abdestillation des Hauptanteils der eingesetzten Lösungsmittel im Vacuum, Sprühtrocknung der Lösung des Alkylenoxid-modifizierten Triazinderivats und gegebenenfalls Nachtrocknung im Vacuum vorzunehmen.

Beispiele für geeignete Triazinderivate, die bei der Herstellung von Polyalkylenoxid-freien Gemischen aus Triazinderivaten mit Hydroxygruppen eingesetzt werden, sind Melamin, Amino-C₁-C₁₂-alkylgruppen substituierte Melamine, Diaminomethyltriazine wie 2,4-Di(6-aminohexylamino)-1,3,5-triazin oder Diaminophenyltriazine, veretherte Methylolmelamine, Ammelin, Ammelid, Melem, Melon, Melam, Benzoguanamin, Acetoguanamin, Methoxymethylbenzoguanamin, Caprinoguanamin und Butyroguanamin.

Bevorzugt wird bei der erfindungsgemässen Herstellung von Polyalkylenoxid-freien Gemischen aus Triazinderivaten mit Hydroxygruppen als Triazinderivat Melamin eingesetzt.

Als weitere Triazinderivate werden bei der erfindungsgemässen Herstellung von Polyalkylenoxid-freien Gemischen aus Triazinderivaten mit Hydroxygruppen Triazinderivate mit C₁-C₈-Alkoholen veretherte Methylolmelamine eingesetzt, wobei 1 bis 5 Wasserstoffatome des Melamins durch C₁-C₈-Alkyloxamethylgruppen substituiert sind.

Bevorzugt eingesetzte Alkylenoxide bei dem erfindungsgemässen Verfahren zur Herstellung von Polyalkylenoxid-freien Gemischen aus Triazinderivaten mit Hydroxygruppen sind Ethylenoxid und/oder Propylenoxid.

Beispiele für geeignete Metallalkyle, die als Katalysatoren bei dem erfindungsgemässen Verfahren zur Herstellung von Polyalkylenoxid-freien Gemischen aus Triazinderivaten mit Hydroxygruppen eingesetzt werden können, sind Lithiumbutyl, Magnesiumdiethyl, Zinkdimethyl, Aluminiumtriisobutyl, Bortriethyl und Titantetrabutyl.

Bevorzugt werden als Metallalkyle C₁-C₄-Metallalkyle von Alkalimetallen, insbesondere Lithiumalkyle.

Beispiele für geeignete Metallhydride, die als Katalysatoren bei dem erfindungsgemässen Verfahren zur Herstellung von Polyalkylenoxid-freien Gemischen aus Triazinderivaten mit Hydroxygruppen eingesetzt werden können, sind Lithiumhydrid und Natriumhydrid.

Als Metallalkoholate werden Metallalkoholate von Alkalimetallen, insbesondere Lithiummethylat, Lithium-tert.butylat, Natriummethylat, Natrium-tert.butylat und/oder Kaliumtert.butylat, als Katalysatoren bei dem erfindungsgemässen Verfahren zur Herstellung von Polyalkylenoxid-freien Gemischen aus Triazinderivaten mit Hydroxygruppen bevorzugt.

Geeignet als Metallalkoholate sind weiterhin Alkoholate von Thioalkoholen wie Lithiumthiomethylat oder Kalium-tert.thiobutylat.

Beispiele für geeignete Metallalkylamide, die als Katalysatoren bei dem erfindungsgemässen Verfahren zur Herstellung von Polyalkylenoxid-freien Gemischen aus Triazinderivaten mit Hydroxygruppen eingesetzt werden können, sind Lithiumdimethylamid, Kaliumdiethylamid und Natriumdiisopropylamid. Weiterhin geeignet sind die homologen Phosphor- Arsen- und Antimonverbindungen wie Lithiumdimethylphosphid oder Natriumdiethylarsenid.

Bei der erfindungsgemässen Herstellung der Triazinderivate wird stets ein Gemisch aus Triazinderivaten gebildet, das frei von Polyalkylenoxid-Nebenprodukten ist. Die Zusammensetzung des Gemischs, d.h. die Anteile an Triazinderivaten a), bei denen die Hydroxyalkylgruppen ausschliesslich als Hydroxyalkylaminogruppen mit den Triazinringen verknüpft sind, und die Anteile an Triazinderivaten b), bei denen die Hydroxyalkylgruppen sowohl als Bis(hydroxyalkyl)iminogruppen als auch als Hydroxyalkylaminogruppen mit den Triazinringen verknüpft sind, wird durch das eingesetzte Verhältnis Alkylenoxid/Triazinderivat bei der Herstellung bestimmt.

Bei niedrigen Alkylenoxidanteilen in der Reaktionsmischung wird ein Triazinderivatgemisch mit hohen Anteilen an Triazinderivaten a), bei denen die Hydroxyalkylgruppen ausschliesslich als Hydroxyalkylaminogruppen mit den Triazinringen verknüpft sind, gebildet.

Bei hohen Alkylenoxidanteilen in der Reaktionsmischung wird ein Triazinderivatgemisch mit niedrigen Anteilen an Triazinderivaten, bei denen die Hydroxyalkylgruppen ausschliesslich als Hydroxyalkylaminogruppen mit den Triazinringen verknüpft sind, und hohen Anteilen an Triazinderivaten b), bei denen die Hydroxyalkylgruppen sowohl als Bis-(hydroxyalkyl)iminogruppen als auch als Hydroxyalkylaminogruppen mit den Triazinringen verknüpft sind, gebildet.

Der Gesamtanteil der in den Triazinderivaten an den Triazinring gebundenen Hydroxyalkylgruppen sowie die Anteile der als Hydroxyalkylaminogruppen bzw. Bis(hydroxyalkyl)iminogruppen kann durch IR-Analyse ermittelt werden. Die einzelnen Spezies der Hydroxyalkylgruppen-substituierten Triazinderivate und deren Anteile im Gemisch aus Triazinderivaten mit Hydroxyalkylaminogruppen und Bis(hydroxyalkyl)iminogruppen lassen sich durch HPLC/MS-Analyse ermitteln.

Die Polyalkylenoxid-freien Gemische aus Triazinderivaten mit Hydroxygruppen sind insbesondere zur Herstellung von Kunststoffen, Flammschutzmitteln, Additiven, Pharmazeutika, Textilhilfsmitteln und Farbstoffen geeignet.

Beispiele für die Herstellung von Kunststoffen, bei denen die Triazinderivate mit Hydroxygruppen als Polyole eingesetzt werden können, sind die Herstellung von Polyurethanen durch Umsetzung mit mehrfunktionellen Isocyanaten, die Herstellung von Polyestem durch Umsetzung mit mehrfunktionellen Carbonsäuren und die Herstellung von Polyhydroxyethern durch Umsetzung mit polyfunktionellen Glycidylverbindungen.

Die Erfindung wird durch nachfolgende Beispiele erläutert :

### Beispiel 1

In einen 20 I Druckreaktor mit Rührer, absteigendem Kühler und Bodenablassventil werden nach dem Spülen mit Inertgas 12 I über Molsieb getrocknetes Dimethylsulfoxid dosiert. Unter intensivem Rühren erfolgt danach die Zugabe von 1,26 kg Melamin, das teilweise gelöst wird. Zu dieser Suspension wird bei Raumtemperatur 300 ml der Katalysatorlösung aus n-Butyllithium in Cyclohexan (1,6 mol/l) zugetropft. Zur Bildung des Lithium-Melamin-Adduktes ist eine Reaktionszeit von 20 min ausreichend. Nachfolgend werden 1,74 kg Propylenoxid in den Druckreaktor dosiert und der Druckreaktor unter Rühren auf 85°C aufgeheizt, wobei sich ein Reaktorinnendruck von 1,2 bar einstellt. Nach 20 min ist das Melamin vollständig gelöst, und nach einer Reaktionszeit von 10 Std. bei 85°C hat sich eine homogene schwach gelblich gefärbte Lösung gebildet, die durch Abdestillation der Hauptmenge des Cyclohexans und Dimethylsulfoxids bei einem Vacuum von 12 mm Hg auf ein Volumen von 6 I eingeengt und durch Sprühtrocknung getrocknet wird.

Die Ausbeute des Gemischs aus Triazinderivaten beträgt, bezogen auf das eingesetzte Propylenoxid, 92%. Die IR-Analyse ergibt für die Anteile der an den Triazinring gebundenen Hydroxypropylgruppen 35 Mol% Hydroxypropylaminogruppen und 65 Mol% Bis(hydroxypropyl)iminogruppen.

### Beispiel 2

In einen 20 I Druckreaktor mit Rührer, absteigendem Kühler und Bodenablassventil werden nach dem Spülen mit Inertgas 13 I über Molsieb getrocknetes Dimethylsulfoxid dosiert. Unter intensivem Rühren erfolgt danach die Zugabe einer Mischung aus 756 g Melamin, 250 g Acetoguanamin und 29 g Dimethylolmelamindibutylether, die teilweise gelöst wird. Zu dieser Suspension wird bei Raumtemperatur 330 ml der Katalysatorlösung aus Isopropyllithium in Cyclohexan (1,5 mol/l) zugetropft. Zur Bildung des Lithium-Adduktes ist eine Reaktionszeit von 20 min ausreichend. Nachfolgend werden 1,16 kg Propylenoxid in den Druckreaktor dosiert und der Druckreaktor unter Rühren auf 85°C aufgeheizt, wobei sich ein Reaktorinnendruck von 1,2 bar einstellt. Nach 15 min ist die Mischung aus Melamin, Acetoguanamin und Dimethylolmelamindibutylether vollständig gelöst, und nach einer Reaktionszeit von 12 Std. bei 85°C hat sich eine homogene schwach gelblich gefärbte Lösung gebildet, die durch Abdestillation der Hauptmenge des Dimethylsulfoxids und Cyclohexans bei einem Vacuum von 12 mm Hg auf ein Volumen von 5 I eingeengt und durch Sprühtrocknung getrocknet wird.

Die Ausbeute des Gemischs aus Triazinderivaten beträgt, bezogen auf das eingesetzte Propylenoxid, 96%. Die IR-Analyse ergibt für die Anteile der an den Triazinring gebundenen Hydroxypropylgruppen 41 Mol% Hydroxypropylaminogruppen und 59 Mol% Bis(hydroxypropyl)iminogruppen.

### Beispiel 3

In einen 20 I Druckreaktor mit Rührer, absteigendem Kühler und Bodenablassventil werden nach dem Spülen mit Inertgas 12 I über Molsieb getrocknetes Dimethylformamid dosiert. Unter intensivem Rühren erfolgt danach die Zugabe einer Mischung aus 1,0 kg Melamin und 490 g Methoxymethylbenzoguanamin, die teilweise gelöst wird. Zu dieser Suspension wird bei Raumtemperatur 500 ml der Katalysatorlösung aus Bortriethyl in n-Heptan (1,9 mol/l) zugetropft. Zur Bildung des Bor- Adduktes ist eine Reaktionszeit von 40 min ausreichend. Nachfolgend werden 1,76 kg Ethylenlenoxid in den Druckreaktor dosiert und der Druckreaktor unter Rühren auf 80°C aufgeheizt, wobei sich ein Reaktorinnendruck von 8 bar einstellt. Nach 30 min ist die Mischung aus Melamin und Methoxymethylbenzoguanamin vollständig gelöst, und nach einer Reaktionszeit von 15 Std. bei 80°C hat sich eine homogene schwach gelblich gefärbte Lösung gebildet, die durch Abdestillation der Hauptmenge des Heptans und des Dimethylformamids bei einem Vacuum von 8 mm Hg auf ein Volumen von 6 I eingeengt und durch Sprühtrocknung getrocknet wird.

Die Ausbeute des Gemischs aus Triazinderivaten beträgt, bezogen auf das eingesetzte Ethylenoxid, 88%. Die IR-Analyse ergibt für die Anteile der an den Triazinring gebundenen Hydroxyethylgruppen 28 Mol% Hydroxyethylaminogruppen und 72 Mol% Bis-(hydroxyethyl)iminogruppen.

### Beispiel 4

In einen 20 I Druckreaktor mit Rührer, absteigendem Kühler und Bodenablassventil werden nach dem Spülen mit Inertgas 12 I über Molsieb getrocknetes Dimethylsulfoxid dosiert. Unter intensivem Rühren erfolgt danach die Zugabe von 1,26 kg Melamin, das teilweise gelöst wird. Zu dieser Suspension wird bei Raumtemperatur 300 ml der Katalysatorlösung aus n-Butyllithium in Cyclohexan (1,6 mol/l) zugetropft. Zur Bildung des Lithium-Melamin-Adduktes ist eine Reaktionszeit von 20 min ausreichend. Nachfolgend werden 3,48 kg Propylenoxid in den Druckreaktor dosiert und der Druckreaktor unter Rühren auf 85°C aufgeheizt, wobei sich ein Reaktorinnendruck von 1,2 bar einstellt. Nach 20 min ist das Melamin vollständig gelöst und nach einer Reaktionszeit von 10 Std. bei 85°C hat sich eine homogene schwach gelblich gefärbte Lösung gebildet, die durch Abdestillation der Hauptmenge des Cyclohexans und Dimethylsulfoxids bei einem Vacuum von 12 mm Hg auf ein Volumen von 6,5 I eingeengt und durch Sprühtrocknung getrocknet wird.

Die Ausbeute des Gemischs aus Triazinderivaten beträgt, bezogen auf das eingesetzte Propylenoxid, 88%. Die IR-Analyse ergibt für die Anteile der an den Triazinring gebundenen Hydroxypropylgruppen 23 Mol% Hydroxypropylaminogruppen und 77 Mol% Bis(hydroxypropyl)iminogruppen.

### Beispiel 5

In einen 20 I Druckreaktor mit Rührer, absteigendem Kühler und Bodenablassventil werden nach dem Spülen mit Inertgas 12 I über Molsieb getrocknetes Dimethylsulfoxid dosiert. Unter intensivem Rühren erfolgt danach die Zugabe von 1,26 kg Melamin, das teilweise gelöst wird. Zu dieser Suspension wird bei Raumtemperatur 350 ml einer Katalysatorlösung, die 40 g Lithium-tert.butylat in Cyclohexan enthält, zugetropft. Nach 30 min. werden 1,8 kg Propylenoxid in den Druckreaktor dosiert und der Druckreaktor unter Rühren auf 90 °C aufgeheizt. Nach 20 min ist das Melamin vollständig gelöst, und nach einer Reaktionszeit von 10 Std. bei 90 °C hat sich eine homogene schwach gelblich gefärbte Lösung gebildet; die durch Abdestillation der Hauptmenge des Cyclohexans und Dimethylsulfoxids bei einem Vacuum von 12 mm Hg auf ein Volumen von 6,5 I eingeengt und durch Sprühtrocknung getrocknet wird.

Die Ausbeute des Gemischs aus Triazinderivaten beträgt 2,9 kg. Die IR-Analyse ergibt für die Anteile der an den Triazinring gebundenen Hydroxypropylgruppen 38 Mol% Hydroxypropylaminogruppen und 62 Mol% Bis(hydroxypropyl)iminogruppen,

### Beispiel 6

In einen 20 I Druckreaktor mit Rührer, absteigendem Kühler und Bodenablassventil werden nach dem Spülen mit Inertgas 12 I über Molsieb getrocknetes Dimethylsulfoxid dosiert. Unter intensivem Rühren erfolgt danach die Zugabe von 1,26 kg Melamin, das teilweise gelöst wird. Zu dieser Suspension wird bei Raumtemperatur 300 ml der Katalysatorlösung, die 54 g Natriummethylat in Cyclohexan enthält, zugetropft. Nach 30 min werden 1,45 kg Propylenoxid in den Druckreaktor dosiert und der Druckreaktor unter Rühren auf 100 °C aufgeheizt. Nach 15 min ist das Melamin vollständig gelöst, und nach einer Reaktionszeit von 3 Std. bei 100 °C hat sich eine homogene schwach gelblich gefärbte Lösung gebildet, die durch Abdestillation der Hauptmenge des Cyclohexans und Dimethylsulfoxids bei einem Vacuum von 12 mm Hg auf ein Volumen von 6 I eingeengt und durch Sprühtrocknung getrocknet wird.

Die Ausbeute des Gemischs aus Triazinderivaten beträgt 2,61 kg. Die IR-Analyse ergibt für die Anteile der an den Triazinring gebundenen Hydroxypropylgruppen 40 Mol% Hydroxypropylaminogruppen und 60 Mol% Bis(hydroxypropyl)iminogruppen.

### Beispiel 7

In einen 20 I Druckreaktor mit Rührer, absteigendem Kühler und Bodenablassventil werden nach dem Spülen mit Inertgas 12 I über Molsieb getrocknetes Dimethylsulfoxid dosiert. Unter intensivem Rühren erfolgt danach die Zugabe von 1,26 kg Melamin, das teilweise gelöst wird. Zu dieser Suspension wird bei Raumtemperatur 320 ml einer Katalysatorlösung, die 56 g Kalium-tert.butylat in Cyclohexan enthält, zugetropft. Nach 30 min werden 1,16 kg Propylenoxid in den Druckreaktor dosiert und der Druckreaktor unter Rühren auf 85 °C aufgeheizt. Nach 20 min ist das Melamin vollständig gelöst, und nach einer Reaktionszeit von 4 Std. bei 85 °C hat sich eine homogene schwach gelblich gefärbte Lösung gebildet, die durch Abdestillation der Hauptmenge des Cyclohexans und Dimethylsulfoxids bei einem Vacuum von 12 mm Hg auf ein Volumen von 6 I eingeengt und durch Sprühtrocknung getrocknet wird.

Die Ausbeute des Gemischs aus Triazinderivaten beträgt 2,33 kg. Die IR-Analyse ergibt für die Anteile der an den Triazinring gebundenen Hydroxypropylgruppen 43 Mol% Hydroxypropylaminogruppen und 57 Mol% Bis(hydroxypropyl)iminogruppen.

## Patentansprüche

1. Polyalkylenoxid-freie Gemische aus Triazinderivaten mit Hydroxygruppen, **dadurch gekennzeichnet, dass** die Gemische aus
a) 20 bis 70 Masse% Triazinderivaten mit Hydroxyalkylaminogruppen der Formel 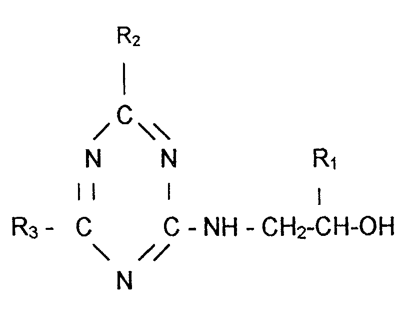
R₁ = H oder C₁-C₄ - Alkyl
R₂ = -H; -OH; C₁-C₄ - Alkyl, -C₆H₅; -NH₂; -NH-CH₂-CHR₁-OH; -NH-CH₂-OR₁ oder -N(-CH₂-OR₁)₂,
R₃ = -H; -OH; C₁-C₄ - Alkyl, -C₆H₅; -NH₂; -NH-CH₂-CHR₁-OH; -NH-CH₂-OR₁ oder -N(-CH₂-OR₁)₂,
und
b) 80 bis 30 Masse% Triazinderivaten mit Bis(hydroxyalkyl)iminogruppen der Formel 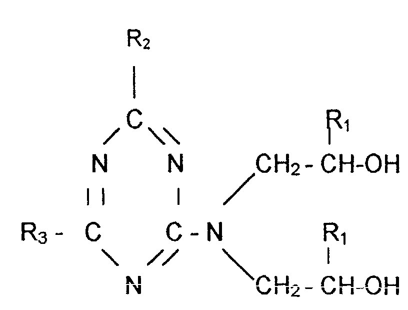
R₁ = H oder C₁-C₄ - Alkyl
R₂ = -H; -OH; C₁-C₄ - Alkyl, -C₆H₅; -NH₂; -NH-CH₂-CHR₁-OH; -N[CH₂-CHR₁-OH]₂, -NH-CH₂-OR₁ oder -N(-CH₂-OR₁)₂,
R₃ = -H; -OH; C₁-C₄ - Alkyl, -C₆H₅; -NH₂; -NH-CH₂-CHR₁-OH; -N[CH₂-CHR₁-OH]₂, -NH-CH₂-OR₁ oder -N(-CH₂-OR₁)₂,
bestehen.

2. Polyalkylenoxid-freie Gemische aus Triazinderivaten mit Hydroxygruppen nach Anspruch 1, **dadurch gekennzeichnet, dass** die Gemische aus
a) Hydroxyalkylaminogruppen substituiertem Triazin mit einem Molverhältnis Triazin/ Hydroxyalkylaminogruppen 1:1 bis 1 : 2,5, und
b) Bis(hydroxyalkyl)iminogruppen und Hydroxyalkylaminogruppen substituiertem Triazin mit einem Molverhältnis Triazin/Hydroxyalkylgruppen 1 : 2,5 bis 1 : 5,5
bestehen.

3. Verfahren zur Herstellung von Polyalkylenoxid-freien Gemischen aus Triazinderivaten mit Hydroxygruppen, **dadurch gekennzeichnet, dass** Gemische aus
a) 20 bis 70 Masse% Triazinderivaten mit Hydroxyalkylaminogruppen der Formel 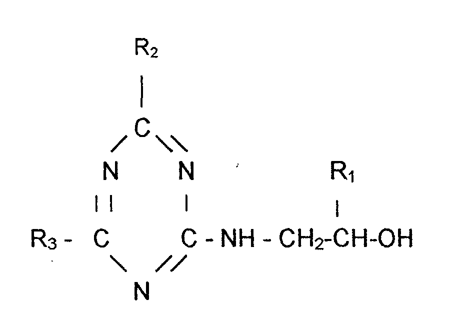
R₁ = H oder C₁-C₄ - Alkyl
R₂ = -H; -OH; C₁-C₄ - Alkyl, -C₆H₅; -NH₂; -NH-CH₂-CHR₁-OH; -NH-CH₂-OR₁ oder -N(-CH₂-OR₁)₂,
R₃ = -H; -OH; C₁-C₄ - Alkyl, -C₆H₅; -NH₂; -NH-CH₂-CHR₁-OH; -NH-CH₂-OR₁ oder -N(-CH₂-OR₁)₂,
und
b) 80 bis 30 Masse% Triazinderivaten mit Bis(hydroxyalkyl)iminogruppen der Formel 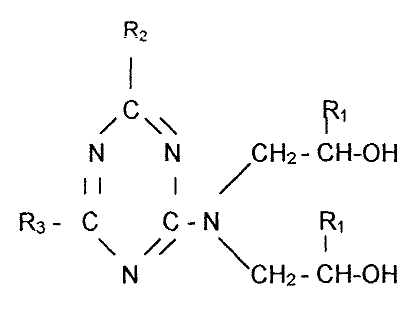
R₁ = H oder C₁-C₄ - Alkyl
R₂ = -H; -OH; C₁-C₄ - Alkyl, -C₆H₅; -NH₂; -NH-CH₂-CHR₁-OH; -N[CH₂-CHR₁-OH]₂, -NH-CH₂-OR₁ oder-N(-CH₂-OR₁)₂,
R₃ = -H; -OH; C₁-C₄ - Alkyl, -C₆H₅; -NH₂; -NH-CH₂-CHR₁-OH; -N[CH₂-CHR₁-OH]₂, -NH-CH₂-OR₁ oder-N(-CH₂-OR₁)₂,
durch katalytische Umsetzung von Alkylenoxiden der Formel 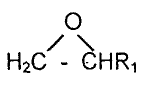 , wobei R₁ = H oder C₁-C₄ - Alkyl bedeuten,
mit Triazinderivaten der Formel 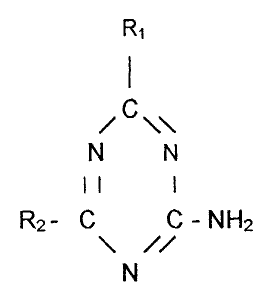
R₁ = -H; -OH; C₁-C₄ Alkyl, -C₆H₅; -NH₂; -NH-CH₂-OR₁ oder -N(-CH₂-OR₁)₂,
R₂ = -H; -OH; C₁-C₄ - Alkyl, -C₆H₅; -NH₂; -NH-CH₂-OR₁ oder -N(-CH₂-OR₁)₂,
bei einem Molverhältnis Triazinderivat/Alkylenoxid 1 : 1,5 bis 1 : 10 in Gegenwart von 0,001 bis 1 Masse%, bezogen auf die Summe von Triazinderivat und Alkylenoxid, Metallalkylen, Metallhydriden, Metallalkoholaten und/oder Metallalkylamiden als Katalysatoren hergestellt werden, wobei die Umsetzung in polaren wasserfreien Lösungsmitteln vom Typ Dimethylsulfoxid, Tetramethylensulfon, N-Methylpyrroli-dinon, Dimethylformamid und/oder Dimethylacetamid bei Reaktionstemperaturen von 35 bis 150°C, Drücken von 1 bis 25 bar und Reaktionszeiten von 1 bis 30 Std. durchgeführt wird.

4. Verfahren zur Herstellung von Polyalkylenoxid-freien Gemischen aus Triazinderivaten mit Hydroxygruppen nach Anspruch 3, **dadurch gekennzeichnet, dass** als Triazinderivat Melamin eingesetzt wird.

5. Verfahren zur Herstellung von Polyalkylenoxid-freien Gemischen aus Triazinderivaten mit Hydroxygruppen nach Anspruch 3, **dadurch gekennzeichnet, dass** als Triazinderivate mit C₁-C₈-Alkoholen veretherte Methylolmelamine eingesetzt werden, wobei 1 bis 5 Wasserstoffatome des Melamins durch C₁-C₈-Alkyloxamethylgruppen substituiert sind.

6. Verfahren zur Herstellung von Polyalkylenoxid-freien Gemischen aus Triazinderivaten mit Hydroxygruppen nach Anspruch 3 bis 5, **dadurch gekennzeichnet, dass** als Alkylenoxide Ethylenoxid und/oder Propylenoxid eingesetzt werden.

7. Verfahren zur Herstellung von Polyalkylenoxid-freien Gemischen aus Triazinderivaten mit Hydroxygruppen nach Anspruch 3, **dadurch gekennzeichnet, dass** als Metallalkyle C₁-C₄-Metallalkyle von Alkalimetallen, insbesondere Lithiumalkyle, eingesetzt werden.

8. Verfahren zur Herstellung von Polyalkylenoxid-freien Gemischen aus Triazinderivaten mit Hydroxygruppen nach Anspruch 3, **dadurch gekennzeichnet, dass** als Metallalkoholate Metallalkoholate von Alkalimetallen, bevorzugt Lithiummethylat, Lithium-tert.butylat, Natriummethylat, Natrium-tert.butylat und/oder Kalium-tert.butylat, eingesetzt werden.

9. Verwendung von Polyalkylenoxid-freien Gemischen aus Triazinderivaten mit Hydroxygruppen zur Herstellung von Kunststoffen, Flammschutzmitteln, Additiven, Pharmazeutika, Textilhilfsmitteln und Farbstoffen.
